# EUROPEAN PATENT APPLICATION

(11) **EP 4 505 868 A1**
(43) Date of publication of application: **12.02.2025**
(21) Application number: 23190737.9
(22) Date of filing: 10.08.2023
(51) Int. Cl.: A01H 1/04

(54) **MOBILE OBSERVATION TRAIL METHOD**

(71) Applicant: KWS SAAT SE & Co. KGaA, 37574 Einbeck (DE)
(72) Inventor: HERBIG, Georg, 97279 Prosselsheim (DE); CZARNECKI, Olaf, 13055 Berlin (DE)
(74) Representative: Michalski Hüttermann & Partner Patentanwälte mbB

(57) **Abstract**

The disclosure refers to an observation trail method for determining a resistance of a plant variety to one or more pest species which infests a field plant of the plant variety, the method comprising
Providing experimental plants of a plant variety (100);
Arranging the experimental plants next to pest-infested field plants at a first point time (150),
wherein the pest-infested field plants are of the same plant variety or another plant variety as the experimental plants and wherein the pest-infested field plants are in a field where field plants of the same or another plant variety is growing,
wherein the pest-infested field plants are infested with a pest species to which resistance of the experimental plants is to be determined;
Classifying a physical state of the experimental plants at a second point of time (400); and
Classifying the physical state of the experimental plants at at least a further point of time being after the second point of time (500).

## Description

### Technical field

The invention refers to an observation trail method for determining a resistance of a plant variety to one or more pest species which infests a field plant of the plant variety.

### Background

Sugar beet root weevil (Asproparthensis punctiventris) belongs to the group of leaf feeding pests in sugar beet and occurrence in sugar beet fields resulting in strong to even fatal damage increased with the ban of neonicotinoid seed treatments for sugar beet in the European Union in 2018. Breeding for herbivory tolerant or even resistant varieties requires reliable methods to observe plant insect interactions. Field observation trials are by far the most valuable methods to observe such interactions, because they typically allow larger quantities of genotypes to be tested and they integrate all possible environmental factors that need to be considered for crop plant breeding. For several years we have tried to monitor interactions between sugar beet genotypes and sugar beet root weevils in regular observation trials in several locations in Germany and Austria, however results were very mixed; often the chosen field sites for planting sugar beet were not or only weakly infested with sugar beet root weevils, while neighbouring fields were heavily attacked. Since there are no available means to predict the occurrence or behaviour of sugar beet root weevil in terms of which fields are going to be infested in spring, successful planning and planting of observation trials is purely random.

### Description of important aspects

The aim is to provide a more reliable, reproducible method for identifying pest-resistant plants.

The task of the invention is solved by the features of the independent claims. Advantageous embodiments are indicated in the dependent claims. If technically possible, the teachings of the dependent claims can be combined as desired with the teachings of the independent and dependent claims.

Accordingly, the task is solved by an observation trail method for determining a resistance of a plant variety to one or more pest species which infests a field plant of the plant variety. Especially, one aspect refers to an observation trail method for determining a resistance of a plant variety to one or more insect species which infests a field plant of the plant variety.

According to a first step, the method comprises providing experimental plants of a plant variety. The experimental plants may be grown in trails. The experimental plants are grown independently of the plants in the field. As an alternative to trails, another means may be used to easily arrange the experimental plants in the field. Experimental plants may be one or several genotypes of one plant variety. The plant variety of the experimental plants may correspond, according to one possibility, to the plant variety of the plants growing in the field. Alternatively or additionally, one or more genotypes of a plant variety may be used which is particularly compatible with the plant variety of the plants growing in the field. Further alternatively or additionally, one or more genotypes of a plant variety which is preferred by the pest to the plant variety of the plants growing in the field may be used.

According to a second step, the method comprises arranging the experimental plants next to pest-infested field plants at a first point time. The pest-infested field plants are of the same plant variety or another plant variety as the experimental plants. The pest-infested field plants are in a field where field plants of the same or another plant variety is growing. The pest-infested field plants are infested with a pest species to which resistance of the experimental plants is to be determined.

The experimental plants are grown at the same time as the plants in the field. Alternatively or additionally, the experimental plants are in a different growth phase than the plants in the field. In particular, the experimental plants in such a case are in an earlier growth phase than the plants in the field. This takes advantage of the fact that young plants are more attractive to pests, especially insects, than adult plants. A mixture of plants with different growth phases is also conceivable. It is conceivable that for each block of test plants that is planted in the field, plants of one growth phase are arranged in the field. Next to means, for example, that the experimental plants are placed adjacent to the row of plants infested with the pests. Alternatively or additionally, the experimental plants may be placed in the row of plants infested with the pests. It is conceivable that for each block of test plants that is planted in the field, plants of one growth phase are arranged in the field.

According to a third step, the method comprises classifying a physical state of the experimental plants at a second point of time. The check of the physical state at the second point of time refers to a check whether the experimental plants are infested with the pest. The second point of time is after the first point of time and can be several days after planting at the first point of time.

According to a fourth step, the method comprises classifying the physical state of the experimental plants at at least a further point of time being after the second point of time. The classification of the physical state at a further point in time refers to an assessment of how much the experimental plants have suffered from the pest. The damage to the experimental plants is classified with a scale. This can be a numerical scale.

Advantageous aspects of the claimed invention are explained below and preferred modified embodiments of the invention are further described below. Explanations, in particular of advantages and definitions of features, are basically descriptive and preferred, but not limiting examples. Where an explanation is limiting, this is expressly mentioned.

The idea of the invention is to place experimental plants at a location at which the desired insects are located, to find out whether the experimental plants are resistant to these pests or not. Accordingly, first, the experimental plants are provided at a separate place from the location where the pests occur. Second, the experimental plants are arranged at the place where the pests have occurred. The plant variety of the experimental plant is to be tested for its resistance to the pest. For this purpose, the experimental plant is not planted in a random location and waited to see whether pests find the plants attractive, as has been the case in the past. Instead, the experimental plant is specifically arranged in a place where the pests have already attacked plants of the same plant variety as the experimental plant or of a different plant variety than the experimental plant. In other words, one of the most important aspects is the shift from stationary observation trials, according to prior art, where the trial location is selected before any pest infestation occurs, to a flexible system where the plants are transported to a field site where the desired pest is present.

For example, the mobile field observation trial method is suitable for to access and phenotype insect damage on sugar beet and other crop plants. Observation trials are a fundamental requirement for all breeding programs because they allow testing of specific or general properties of breeding material under natural conditions. The observation trials are planted in fields and plants are exposed to environmental conditions in that location. It is one of the big challenges in insect resistance breeding in crops, such as sugar beet, , that occurrence of insects in fields is unpredictable. Plantations in field may or may not be invaded by the target insect depending on currently unknown factors; insect population development may be on of these. Moving from fixed locations to mobile observation trials overcomes that limitation. Experimental plants are not sown in soil anymore but pre-cultivated to the desired stage in pots or other transportable vessels and then transported to field sites where presence of the target insect has been verified by observation. After transferring them to the field site, insect damage can be monitored and phenotyped properly.

Observation trials are a fundamental requirement for resistance breeding in sugar beet and probably all field crops. The growing field of insect resistance breeding depends on such trials but deals with highly mobile target pests in contrast to, e.g., fungal pests. Currently, no tools or models are available to predict occurrence or infestation density of any target insect in a specific field site that would allow to choose such a field site before transplanting. In fact, it is common practice to plant observation trials in a set of potential locations hoping that one or two of them eventually are infested by the target insect. A high number of testing locations is not only negatively impacting workload and costs of a breeding program, but it also requires high seed amounts of early breeding material which is very often limited and providing enough seed has major impact on the design of resistance breeding programs. Moving from fixed locations to mobile observation trials where insect or pest occurrence, respectively can be predicted helps to overcome these limitations. Moreover, mobile observation trials ensure a steady and annual selection process which is a requirement development of breeding material. Thus, the present object is essentially focused on insects. The method does not change with different insects.

The present object is essentially useful to determine resistance of plants with respect to Pentastiridius leporinus (reed glass-winged cicada). This insect triggers Syndrome Basses Richesses (SBR), that means a syndrome of low sugar levels of sugar beets. This disease is becoming more and more relevant. It is caused by two plant pathogenic bacteria and transmitted by a cicada. Symptoms of SBR are yellowed, older leaves, lanceolate or asymmetrically shaped, younger leaves, and necrotic browned vascular bundles in the beet body.

According to a modified embodiment, the method comprises the following steps:
According to a first step, the method comprises providing experimental plants of a plant variety. The experimental plants may be grown in trails. The experimental plants are grown independently of the plants in the field. As an alternative to trails, another means may be used to easily arrange the experimental plants in the field. Experimental plants may be one or several genotypes of one plant variety. The plant variety of the experimental plants may correspond, according to one possibility, to the plant variety of the plants growing in the field. Alternatively or additionally, one or more genotypes of a plant variety may be used which is particularly compatible with the plant variety of the plants growing in the field. Further alternatively or additionally, one or more genotypes of a plant variety which is preferred by the pest to the plant variety of the plants growing in the field may be used.

According to a second step, the method comprises identifying pest-infested field plants of the same plant variety or another plant variety in a field where field plants of the same or another plant variety is growing. The pest-infested field plants are infested with a pest species to which resistance of the experimental plants is to be determined. The experimental plants are grown at the same time as the plants in the field. Alternatively or additionally, the experimental plants are in a different growth phase than the plants in the field. In particular, the experimental plants in such a case are in an earlier growth phase than the plants in the field. This takes advantage of the fact that young plants are more attractive to pests, especially insects, than adult plants. A mixture of plants with different growth phases is also conceivable. It is conceivable that for each block of test plants that is planted in the field, plants of one growth phase are arranged in the field.

The step of identifying pest-infected field plants may be realized with the help of drones, for example. In this case, pest-infested areas in the field are determined with the help of an algorithm that detects bare patches in the field or changes in the colour of the leaves. Alternatively, identifying and/or recording of conditions of plants in fields with drones and small robots may be conceivable. When the plants are in the field, a mobile mast, for example with a height of three meters in maximum with an RGB camera is set up. This ensures that many individual plants can be observed.

According to a third step, the method comprises arranging the experimental plants next to the pest-infested field plants at a first point time. Next to means, for example, that the experimental plants are placed adjacent to the row of plants infested with the pests. Alternatively or additionally, the experimental plants may be placed in the row of plants infested with the pests. It is conceivable that for each block of test plants that is planted in the field, plants of one growth phase are arranged in the field.

According to a fourth step, the method comprises classifying a physical state of the experimental plants at a second point of time. The check of the physical state at the second point of time refers to a check whether the experimental plants are infested with the pest. The second point of time is after the first point of time and may be several days after planting at the first point of time.

According to a fifth step, the method comprises classifying the physical state of the experimental plants at at least a further point of time being after the second point of time. The classification of the physical state at a further point in time refers to an assessment of how much the experimental plants have suffered from the pest. The damage to the experimental plants is classified with a scale. This may be a numerical scale.

According to a modified embodiment, the method comprises the following steps:
According to a first step, the method comprises providing experimental plants of a plant variety. The experimental plants are grown in trails. The experimental plants are grown independently of the plants in the field. As an alternative to trails, another means may be used to easily arrange the experimental plants in the field. Experimental plants may be one or several genotypes of one plant variety. The plant variety of the experimental plants may correspond, according to one possibility, to the plant variety of the plants growing in the field. Additionally, one or more genotypes of a plant variety may be used which is particularly compatible with the plant variety of the plants growing in the field. Further additionally, one or more genotypes of a plant variety which is preferred by the pest to the plant variety of the plants growing in the field may be used.

According to a second step, the method comprises identifying pest-infested field plants of the plant variety in a field where field plants of the same plant variety is growing. The pest-infested field plants are infested with a pest species to which resistance of the experimental plants is to be determined. In other words, the idea is that now the places where the pests, especially insects, are present, are specifically searched for. Which plants are selected by pests under which circumstances cannot be predicted in a reproducible way. The fact that a pest, in particular an insect, has attacked a certain plant variety at a certain location is therefore used. The experimental plants are then installed at this location.

According to a third step, the method comprises arranging the experimental plants next to the pest-infested field plants at a first point time.

According to a fourth step, the method comprises classifying a physical state of the experimental plants at a second point of time.

According to a fifth step, the method comprises classifying the physical state of the experimental plants at at least a further point of time being after the second point of time.

It is preferred that the sequence of method steps may be varied, unless technically required in an explicit sequence. However, the aforementioned sequence of method steps is particularly preferred.

### Brief description of aspects of the claims

In the following, aspects of the claims are explained in more detail with reference to the accompanying figures using preferred embodiments. The formulation Figure is abbreviated as Fig.

The figures show
- Fig. 1a: a schematic view of a field with plants of one plant variety, some plants being infested with a pest;
- Fig. 1b: a schematic view of the field, wherein experimental plants are arranged at two blocks next to infested plants;
- Fig. 1c: a schematic view of the field, wherein some of the experimental plants are infested;
- Fig. 1d: a schematic view of the field, wherein some of the experimental plants are infested and harmed by the pest species;
- Fig. 2: a flowchart with process steps according to an embodiment; and
- Fig. 3: a flowchart with process steps according to another embodiment.

### Detailed description of the embodiments

The described embodiments are merely examples that can be modified and/or supplemented in many ways within the scope of the claims. Any feature described for a particular embodiment may be used independently or in combination with other features in any other embodiment. Any feature described for an embodiment of a particular claim category may also be used in a corresponding manner in an embodiment of another claim category.

For the sake of comprehensibility, some of the components described in the figures are always marked with reference signs and not all components.

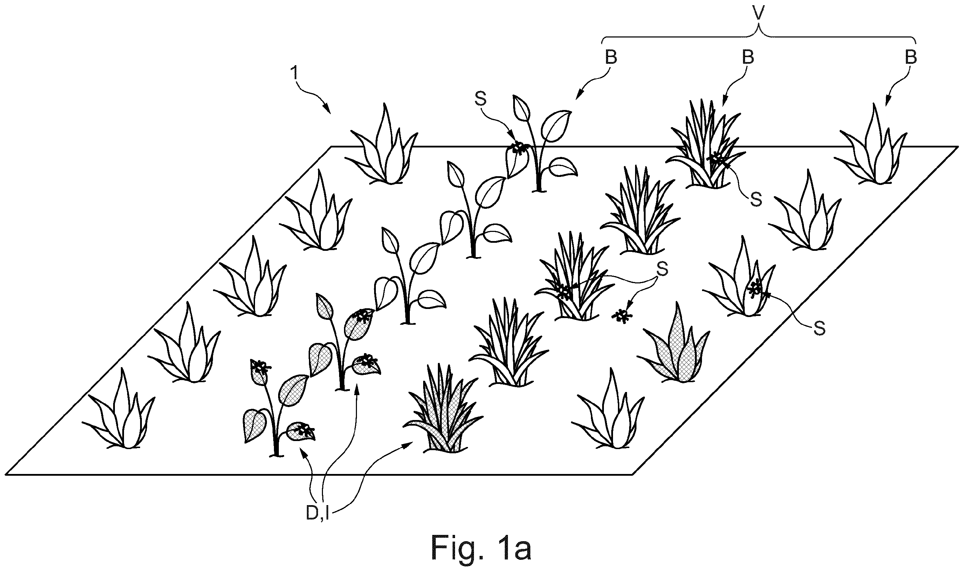

Figure 1a shows a schematic view of a field 1 with plants B of one plant variety V, some plants B being infested with a pest species S. Schematically, a few beetles are shown on the plants B. Some plants B have already been harmed by the beetles, marked with the reference sign D as an example. The field 1 with the pests was specifically searched for.

The harmfulness of the pests in the field 1 for experimental plants A is to be tested. The experimental plants A are therefore only planted at a time when field 1 is already infested with pests.

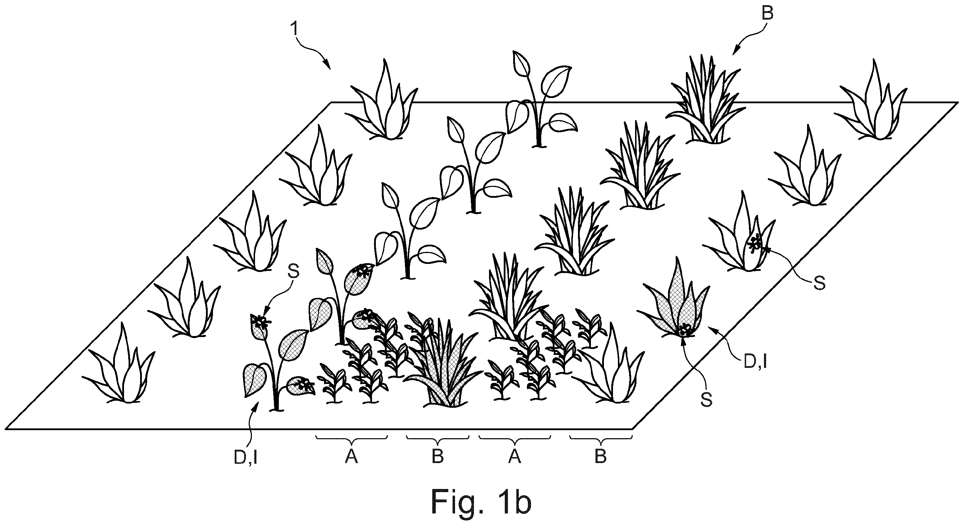

Figure 1b shows a schematic view of the field 1, wherein experimental plants A are arranged at two blocks next to infested plants I. At a first point of time, the experimental plants A are planted in blocks between the rows of plants B that are infested with the pest S. The experimental plants A are planted in trays, which are not shown in the figure. The experimental plants A were previously grown in a place other than the field 1. The other place may be a greenhouse, for example. The plants A are watered once after planting. The experimental plants A may be planted within the planting rows or next to the planting rows. The experimental plants A have the same age as the plants B of the field 1. That means, the experimental plants A have been sown parallel to the plants B of the field 1 at the same point of time.

Alternatively, the experimental plants A may be in an earlier growth stage than the plants B, I in the field 1. Younger plants may be more attractive to the pest and so the likelihood of the experimental plants A being considered by the pest may be increased.

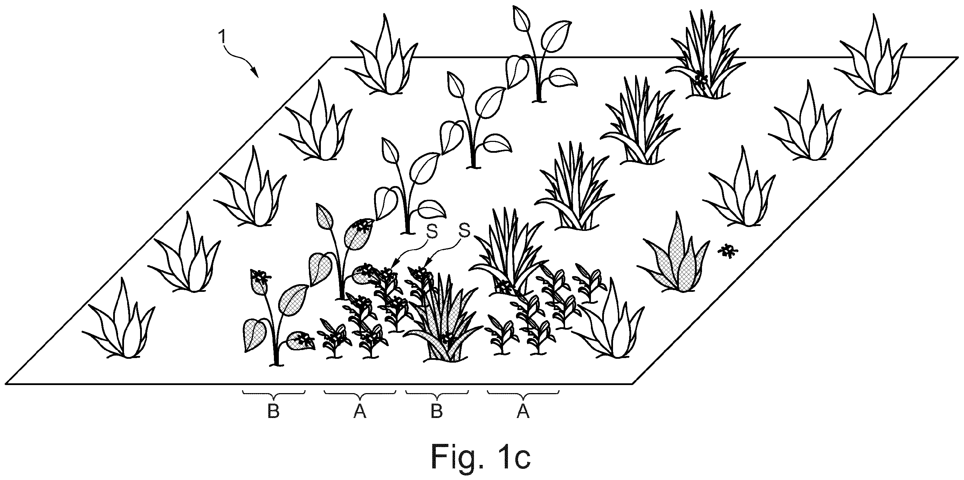

Figure 1c shows a schematic view of the field 1, wherein some of the experimental plants A are infested. Figure 1c thus shows the field 1 at a later point in time after planting the experimental plants A. This later point of time may be one week after having planted the experimental plants A. At this point of time, it is verified that the experimental plants A are infected with the pest species S.

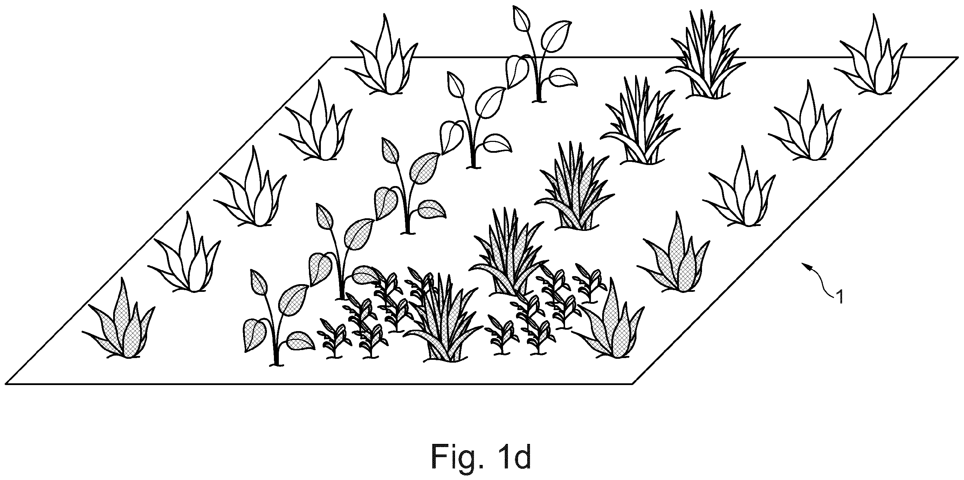

Figure 1d shows a schematic view of the field 1, wherein some of the experimental plants A are infested and harmed by the pest species S.

Field tests using the method described above was carried out, for example, for assessing sugar beet root weevil damage in sugar beet genotypes. For example, 50 sugar beet genotypes were sown in soil in the greenhouse in April to parallel the sugar beet sowing time in the sugar beet growing target region in Eastern Austria. 80 plants per genotype were pricked in two transportable 40-well trays and acclimated to outdoors climate in mid of April. Meanwhile local sugar beet root weevil occurrence situation was closely observed. One field was identified by presence of sugar beet root weevils and trays have been moved to this field in the last week of April. Trays were buried in the chosen field in two blocks of 50 trays each, where one block represented a randomized set of 50 genotypes. Trays were watered once. Presence of sugar beet root weevils was verified after 7 days (see also Figure 1c) and herbivory damage was rated on a 1 to 9 scale after two weeks of field exposition (see Figure 1d). Trays and plants have been removed from the field afterwards.

The principle of the observation trail method described above may also be applied to other plant varieties and/or to investigate pest resistance of experimental plants to other pests. The pest species S, which pest-infests the field plants B, is an insect, in particular a weevil.

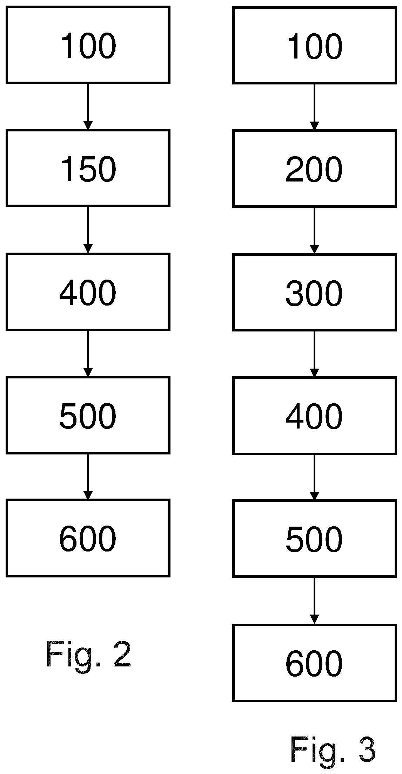

Figure 2 shows a flowchart with process steps according to an embodiment. The flowchart comprises the method steps of an observation trail method for determining a resistance of a plant variety V1, V2 to one or more pest species S which infests a field plant B of the plant variety V1, V2. In other words, plants of a plant variety can grow in the field. Cultivation with a mixed crop is also conceivable.

The method comprises, according to a step indicated by reference number "100", providing experimental plants A of a plant variety V1. The plant variety of the experimental plant A is to be tested for its resistance to the pest. for this purpose, the experimental plant A is not planted in a random location and waited to see whether pests find the plants A attractive, as has been the case in the past. Instead, the experimental plant A is specifically arranged in a place where the pests have already attacked plants of the same plant variety V1 as the experimental plant A or of a different plant variety V2 than the experimental plant A.

The method comprises, according to a step indicated by reference number "150", arranging the experimental plants A next to pest-infested field plants I at a first point of time.

The pest-infested field plants I are of the same plant variety V1 or of another plant variety V2 as the experimental plants A. The pest-infested field plants I are in a field 1 where field plants B of the same plant variety V1 or of another plant variety V2 is growing. The pest-infested field plants I are infested with a pest species S to which resistance of the experimental plants A is to be determined.

The method comprises, according to a step indicated by reference number "400", classifying a physical state of the experimental plants A at a second point of time. The second point of time lies behind the first point of time. For example, the second point of time can be several days/one week after the first point of time.

Finally, the method comprises, according to a step indicated by reference number "500", classifying the physical state of the experimental plants A at at least a further point of time being after the second point of time.

Figure 3 shows a flowchart with process steps according to an embodiment. The method is an observation trail method for determining a resistance of a plant variety V to one or more pest species S which infests a field plant B of the plant variety V.

The method comprises the following steps:
According to step "100", the method comprises providing experimental plants A of a plant variety V. Providing experimental plants A of the plant variety V comprises providing a plurality of plants of a at least a first kind of plant genotype G1 and a second kind of plant genotype G2, wherein each of the plant genotypes G1, G2 belongs to the plant variety V. Moreover, providing experimental plants A of a plant variety V comprises growing the experimental plants A in a greenhouse. The experimental plants A are sown at the same point of time than the plants B, I of the field 1; or the experimental plants A are in an earlier growth stage than the plants B, I. The plants may be placed in a randomized design, with min. four rows and four columns and min. 0.5 m distance between the rows, for example.

According to step "200", the method comprises identifying pest-infested field plants I of the plant variety V in a field 1 where field plants B of the same plant variety V is growing. The pest-infested field plants I are infested with a pest species S to which resistance of the experimental plants A is to be determined.

According to step "300", the method comprises arranging the experimental plants A next to the pest-infested field plants I at a first point time. Arranging the experimental plants A next to the pest-infested field plants I comprises planting the experimental plants A between the pest-infested field plants I and next to the pest-infested field-plants I; in particular planting the experimental plants in planting rows of the pest-infested plants and/or planting the experimental plants between planting rows of the pest-infested plants. After planting the experimental plants A between the pest-infested field plants I and next to the pest-infested field-plants I the experimental plants A are watered. Furthermore, arranging the experimental plants A next to the pest-infested field plants I comprises arranging the experimental plants A in at least one block, in particular at least one tray, in the field; or arranging the experimental plants A in at least two blocks, in particular at least one tray in each of the blocks, in the field, wherein the blocks have a distance to each other being the radius of movement or spread of the pest species S infesting the pest-infested plants I. In addition, arranging the experimental plants A between the pest-infested field plants I at a first point time comprises transporting the experimental plants A to the pest-infested field plants I.

According to step "400", the method comprises classifying a physical state of the experimental plants A at a second point of time. Classifying the physical state of the experimental plants A at a second point of time comprises, according to step "400A", verifying that the experimental plants A are infested with the pest species S.

According to step "500", the method comprises classifying the physical state of the experimental plants A at at least a further point of time being after the second point of time. Classifying the physical state of the experimental plants A at at least the further point of time being after the second point of time comprises rating herbivory damage of the experimental plants A on a, particularly numeric, leaf damage, LD, rating scale with LD rating values. The leaf damage rating values are given as means of both of all experimental plants A arranged on the field, either as absolute values for each of the experimental plants A or as percentage deviation values from a mean value of the damage rating values of all the experimental plants A.

According to step "600", the method comprises removing the experimental plants A from the field after classifying the physical state of the experimental plants A.

### Reference signs

- 1: field

- A: experimental plant
- B: plants of a plant variety
- D: harmed plants
- I: infested plants
- S: pest species/beetle/weevil
- V: plant variety

## Claims

1. An observation trail method for determining a resistance of a plant variety (V1, V2) to one or more pest species (S) which infests a field plant (B) of the plant variety (V1, V2), the method comprising
Providing experimental plants (A) of a plant variety (V1) (100);
Arranging the experimental plants (A) next to pest-infested field plants (I) at a first point time (150, 300),
wherein the pest-infested field plants (I) are of the same plant variety (V1) or another plant variety (V2) as the experimental plants (A) and wherein the pest-infested field plants (I) are in a field (1) where field plants (B) of the same (V1) or another plant variety (V2) is growing,
wherein the pest-infested field plants (I) are infested with a pest species (S) to which resistance of the experimental plants (A) is to be determined;
Classifying a physical state of the experimental plants (A) at a second point of time (400); and
Classifying the physical state of the experimental plants (A) at at least a further point of time being after the second point of time (500).

2. The method of claim 1, the method comprising
Arranging the experimental plants (A) next to pest-infested field plants (I) at a first point time (150, 300),
wherein the pest-infested field plants (I) are of the same plant variety (V1) as the experimental plants (A) and wherein the pest-infested field plants (I) are in a field (1) where field plants (B) of the same plant variety (V1) is growing,
wherein the pest-infested field plants (I) are infested with a pest species (S) to which resistance of the experimental plants (A) is to be determined.

3. The method of claim 1 or 2, the method comprising
Providing experimental plants (A) of a plant variety (V1) (100);
Identifying pest-infested field plants (I) of the plant variety (V) in a field (1) where field plants (B) of the same plant variety (V1) or another plant variety (V2) is growing (200), wherein the pest-infested field plants (I) are infested with a pest species (S) to which resistance of the experimental plants (A) is to be determined; and, thereafter,
Arranging the experimental plants (A) next to pest-infested field plants (I) at the first point time (300).

4. The method of any one of the preceding claims, comprising
Classifying the physical state of the experimental plants (A) at a second point of time (400) comprises
Verifying that the experimental plants (A) are infested with the pest species (S) (400A).

5. The method of any one of the preceding claims, wherein
Classifying the physical state of the experimental plants (A) at at least the further point of time being after the second point of time (500) comprises
Rating herbivory damage of the experimental plants (A) on a, particularly numeric, leaf damage, LD, rating scale with LD rating values,
wherein particularly the leaf damage rating values are given as means of both of all experimental plants (A) arranged on the field (1), either
as absolute values for each of the experimental plants (A) or
as percentage deviation values from a mean value of the damage rating values of all the experimental plants (A).

6. The method of any one of the preceding claims, comprising
Removing the experimental plants (A) from the field (1) after classifying the physical state of the experimental plants (A) (600).

7. The method of any one of the preceding claims, wherein
Arranging the experimental plants (A) next to the pest-infested field plants (I) (300) comprises
Planting the experimental plants (A) between the pest-infested field plants (I) and next to the pest-infested field-plants (I); in particular planting the experimental plants (A) in planting rows of the pest-infested plants (I) and/or planting the experimental plants (A) between planting rows of the pest-infested plants (I).

8. The method of the preceding claim, wherein
after planting the experimental plants (A) between the pest-infested field plants (I) and next to the pest-infested field-plants (I) the experimental plants (A) are watered.

9. The method of any one of the preceding claims, wherein
Arranging the experimental plants (A) next to the pest-infested field plants (I) comprises
Arranging the experimental plants (A) in at least one block, in particular at least one tray, in the field (1); or
Arranging the experimental plants (A) in at least two blocks, in particular at least one tray in each of the blocks, in the field (1), wherein the blocks have a distance to each other being the radius of movement or spread of the pest species (S) infesting the pest-infested plants (I).

10. The method of any one of the preceding claims, wherein
Providing experimental plants (A) of the plant variety (V1, V2) (100) comprises
Providing a plurality of plants of a at least a first kind of plant genotype (G1) and a second kind of plant genotype (G2), wherein each of the plant genotypes (G1, G2) belongs to the plant variety (V1, V2).

11. The method of any one of the preceding claims, wherein
the pest species (S), which pest-infests the field plants (B), is an insect, in particular a weevil.

12. The method of any one of the preceding claims, wherein
Providing experimental plants (A) of the plant variety (V1, V2) (100) comprises
Growing the experimental plants (A) in a greenhouse.

13. The method of any one of the preceding claims, wherein
Arranging the experimental plants (A) between the pest-infested field plants (I) at a first point time (300) comprises
Transporting the experimental plants (A) to the pest-infested field plants (I).

14. The method of any one of the preceding claims, wherein
the experimental plants (A) are sown at the same point of time than the plants (B, I) of the field (1); or
the experimental plants (A) are in an earlier growth stage than the plants (B, I).
